(19) Europäisches Patentamt
European Patent Office
Office européen des brevets

(11) EP 0 544 162 B1

(12) EUROPÄISCHE PATENTSCHRIFT

(45) Veröffentlichungstag und Bekanntmachung des Hinweises auf die Patenterteilung:
03.01.1996 Patentblatt 1996/01

(51) Int. Cl.$^6$: **C07C 68/00**, C07C 69/96

(21) Anmeldenummer: 92119437.9

(22) Anmeldetag: 13.11.1992

(54) **Verfahren zur Herstellung von Dialkylcarbonaten**

Process for the preparation of dialkyle carbonates

Procédé de préparation de carbonates de dialkyle

(84) Benannte Vertragsstaaten:
BE DE ES FR GB IT NL

(30) Priorität: 26.11.1991 DE 4138755

(43) Veröffentlichungstag der Anmeldung:
02.06.1993 Patentblatt 1993/22

(73) Patentinhaber: BAYER AG
D-51368 Leverkusen (DE)

(72) Erfinder:
• Kricsfalussy, Zoltan, Dr.
W-5090 Leverkusen 1 (DE)
• Waldmann, Helmut, Dr.
W-5090 Leverkusen 1 (DE)
• Traenckner, Hans-Joachim, Dr.
W-5090 Leverkusen 1 (DE)
• Zlokarnik, Marko, Prof. Dr.
W-5000 Köln 80 (DE)
• Schomäcker, Reinhard, Dr.
W-5090 Leverkusen 3 (DE)

(56) Entgegenhaltungen:
• CHEMICAL ABSTRACTS, vol. 91, no. 9 Columbus, Ohio, US; abstract no. 74204v, ITATANI HIROSHI 'CARBONIC ACID ESTERS' Seite 559 ;Spalte L ; & JP-A-54 024 827

EP 0 544 162 B1

**Beschreibung**

Die Erfindung betrifft ein verbessertes Verfahren zur Herstellung von Dialkylcarbonaten.

Dialkylcarbonate, insbesondere Dimethylcarbonat, sind Zwischenprodukte mit geringer Toxizität und können giftige Zwischenprodukte, wie Phosgen oder Dimethylsulfat, bei vielen Reaktionen ersetzen. Es ist ferner nicht korrosiv. Bei seiner Verwendung entstehen keine umweltschädlichen Nebenprodukte.

Beispiele solcher Reaktionen der Dialkylcarbonate sind die Herstellung von Urethanen aus aliphatischen oder aromatischen Aminen, die wiederum zu den entsprechenden Isocyanaten gespalten werden können. Dimethylcarbonat kann beispielsweise auch Dimethylsulfat bei der Quaternisierung von Aminen oder bei der Methylierung von Phenol oder von Naphtholen ersetzen. Dimethylcarbonat kann ferner dem Fahrbenzin als Oktanzahl-Verbesserer zugesetzt werden, z.B. anstelle von Bleiverbindungen. Angesichts dieser Bedeutung der Dialkylcarbonate fehlt es immer noch an einem technisch einfachen und umweltverträglichen Produktionsverfahren, das für große Kapazitäten ohne wesentliche Nebenproduktbildung oder gekoppelte Stoffkreisläufe geeignet ist.

Zur Herstellung von Dialkylcarbonaten gibt es verschiedene, im kleinen Maßstab bereits auch technisch erprobte Herstellungsverfahren. Die Herstellungswege, die auf der katalytischen Umsetzung von Alkanolen mit Kohlenmonoxid und Sauerstoff gemäß nachstehender Gleichung beruhen, sind von verschiedenen Arbeitsgruppen intensiv bearbeitet worden:

$$2 \ ROH + CO + 1/2 \ O_2 \xrightarrow[\text{salze}]{\text{Kupfer-}} RO\text{-}CO\text{-}OR + H_2O$$

So hat man die als Katalysator wirkenden Kupferverbindungen in Form verschiedener Kupfersalze eingesetzt. Bei der Verwendung von Kupfer-II-chlorid als Katalysator gemäß JP-45/11129 (1970) erreicht man unbefriedigende Selektivitäten. Störend ist vor allem die Bildung größerer Mengen an Methylchlorid, das wegen seiner großen Flüchtigkeit dazu neigt, sich ubiquitär in der ganzen Produktionsanlage zu verteilen und praktisch in der gesamten Anlage zu Korroionen führen kann.

Bessere Selektivität erhält man bei der Verwendung von organischen Komplexbildnern (DE-A 2110194), aber dort hat man das Problem der Abtrennung der Katalysatorsalze, die im Reaktionsgemisch teils gelöst sind, zum größeren Teil aber als Suspension vorliegen.

Ganz besonders problematisch ist die Durchführung dieser Reaktion gemäß DE-A 2743 690, da die Katalysatorsalze im Reaktionsgemisch praktisch vollständig ungelöst, sondern lediglich suspendiert sind. Diese Salze müssen durch die Reaktionszone und durch die Kühlaggregate gefördert und nach der Reaktion mechanisch, z.B. mit Hilfe von Zentrifugen, abgetrennt werden. Dies bewirkt neben der bereits erwähnten Korrosion auch Erosionen, schlechten Wärmeübergang sowie Verstopfungen und Verkrustungen.

Um diese Nachteile eines Katalysatorkreislaufs zu vermeiden, hat man Vorschläge gemacht, die Katalysatorsalze im Reaktor suspendiert stationär zu halten und Methanol, CO und Sauerstoff in den Reaktor einzudosieren, während man aus dem Reaktor das gebildete Dialkylcarbonat und das Reaktionswasser zusammen mit im Überschuß eingesetzten Methanol abdestilliert (EP 0 413 215 A2). Dabei besteht das flüssige Reaktionsmedium im wesentlichen aus dem umzusetzenden Alkanol (EP-0 413 215, Seite 3, Zeile 52), so daß das Molverhältnis zwischen Alkanol und Cu-Salz sehr hoch ist (bevorzugt 1 : 0,01-0,05). Dies hat den Nachteil, daß die Reaktionsgeschwindigkeit relativ niedrig ist. Problematisch dabei ist auch die Notwendigkeit, eine niedrige Dialkylcarbonat-Konzentration einzustellen. Dies ist nicht leicht, da die Reaktion bei hohem Systemdruck durchgeführt wird und die Löslichkeiten von Dialkylcarbonat und auch von Wasser im Reaktionsmedium, das im wesentlichen aus Methanol besteht, sehr hoch sind. Dies bedeutet, daß die Abtrennungen von Dialkylcarbonat und Wasser mit einer relativ großen Inertgas- beziehungsweise Methanolgasmenge erzwungen werden muß.

Im übrigen ist auch bei diesem Vorschlag damit zu rechnen, daß nach einiger Zeit der Katalysator ausgetauscht, beziehungsweise ständig zu einem bestimmten Anteil erneuert werden muß, was mit dem eingangs erwähnten Problem der Katalysatorabtrennung, -regeneration und -rückführung verbunden ist.

Demgegenüber wurde nun gefunden, daß man ohne diese Probleme die Umsetzung von $C_1$-$C_4$-Alkanolen mit Kohlenmonoxid und Sauerstoff zu $C_1$-$C_4$-Dialkylcarbonaten durchführen kann, wenn man $C_1$-$C_4$-Alkanol, CO und $O_2$ in einer Cusalz enthaltende Salzschmelze bei 120-300°C, bevorzugt bei 120-180°C, besonders bevorzugt bei 120-150°C umsetzt.

Als Alkanol kommen Methanol, Ethanol, Propanol, Isopropanol, n-Butanol und sec-Butanol in Frage. Bevorzugt sind Methanol und Ethanol, besonders bevorzugt ist Methanol.

Die Reaktion wird in einer Cu-salze enthaltenden Salzschmelze durchgeführt, wobei als Cu-salze Cu-I- und Cu-II-verbindungen sowie Gemische davon in Frage kommen, Grundsätzlich sind alle bekannten Cu-salze, sofern sie in der Salzschmelze nur einigermaßen löslich sind, geeignet.

Geeignet sind neben den Halogeniden, wie z.B. den Chloriden oder den Bromiden, auch die Cyanide, Rhodanide, Sulfate, Nitrate, Carbonate, Acetate, Formiate, Oxalate, Alkoholate, z.B. Cu-methoxychlorid. Cu kann auch in Form

2

EP 0 544 162 B1

komplexierter Verbindungen, wie den Acetylacetonaten, oder Cu-N-komplexen, wie Cu-Pyridin- oder auch Cu-Dipyridylkomplexen, eingesetzt werden.

Für die Salzschmelze nimmt man im allgemeinen Gemische von Salzen, die einen niedrigen Schmelzpunkt haben, das heißt, die ein Eutektikum bilden. Es ist daher vorteilhaft, Mengenverhältnisse der Salze entsprechend der Zusammensetzung des Eutektikums zu verwenden. Solche Eutektika können von Cu-salzen untereinander oder von Cu-salzen und weiteren Salzen gebildet werden.

Neben den Cu-salzen kann man daher im Prinzip alle chemisch inerten, oder auch im erfindungsgemäßen Sinne katalytisch wirksamen, das heißt, die Aktivierungsenergie für die Oxycarbonylierung von Alkanolen herabsetzenden Salze verwenden- Neben Cu-salzen kann man dabei eine breite Anzahl von Salzen oder salzartigen Verbindungen verwenden. In der Regel setzt man Gemische von Cu-salzen und solchen salzartigen Verbindungen ein. Bevorzugt setzt man die Halogenide der 1.-3. Haupt- und Nebengruppen ein. Besonders geeignet sind die Chloride der Alkalimetalle, wie NaCl oder KCl, oder Chloride der Erdalkalien, wie $CaCl_2$ oder $MgCl_2$, sowie $ZnCl_2$. Aber auch die Verwendung von weniger häufigen Verbindungen wie die Chloride von Thallium, Indium oder Gallium ist möglich.

Eine gut geeignete Schmelze besteht beispielsweise aus Cu-I-Chlorid und KCl in unterschiedlichen Mengenverhältnissen. Im allgemeinen wählt man Mischungen mit einem hohen Gehalt an Cu-Verbindungen z.B. ein Gewichtsverhältnis von Cu-I-Chlorid zu KCl von 60 - 70 zu 40 - 30.

Die Reaktionstemperatur beträgt im allgemeinen 120°C bis 300°C, bevorzugt 120°C bis 180°C, typische Reaktionstemperaturen liegen bei 120°C-150°C.

Die Reaktion kann bei Normaldruck durchgeführt werden. Um eine genügend hohe Reaktionsgeschwindigkeit zu erhalten, ist es jedoch zweckmäßig, bei erhöhtem Druck, z.B. bei 5-50 bar, bevorzugt bei 10-30 bar, besonders bevorzugt bei 15-25 bar, zu arbeiten.

Die Molverhältnisse der zum Einsatz gebrachten Reäktionskomponenten sind für die Reaktionsgeschwindigkeit und für die Selektivität der Reaktion von Bedeutung. Als Nebenprodukte treten bei Nichtbeachtung dieser Zusammenhänge beispielsweise das Dimethylacetal des Formaldehydes oder - was besonders störend ist - Methylchlorid auf.

Im allgemeinen wählt man einen molaren Überschuß von Methanol zu Kohlenmonoxid und wiederum einen Überschuß von Kohlenmonoxid zu Sauerstoff, höchstens jedoch molare Mengen von CO bzw. $O_2$. So wählt man Molverhältnisse von Alkanol zu CO und $O_2$ von 1: (1-0,01) : (1-0,01), bevorzugt von 1 : (0,5-0,02) : (0,3-0,02). Dabei resultieren ein Methanolumsatz von beispielsweise 10 bis 50 % und ein CO-Umsatz von 10 - 80 %. Sauerstoff wird im allgemeinen völlig umgesetzt. Bei der Mengendosierung müssen selbstverständlich die Explosionsgrenzen beachtet werden. Gegebenenalls kann in Gegenwart von Inertgasen, wie $N_2$ oder $CO_2$, gearbeitet werden.

Es ist jedoch gleichfalls möglich, mit einem molaren Überschuß an CO gegenüber Methanol zu arbeiten. So kann man beispielsweise ein Molverhältnis von Alkanol:CO von 1:1-50 wählen.

Einegut geeignete Mischung ist bei einem Molverhältnis von 1:15 gegeben.

Der Sauerstoff kann beispielsweise in Form von atmosphärischer Luft oder von mit $O_2$ angereicherter Luft eingesetzt werden.

Die nicht umgesetzten Anteile von Methanol und CO können nach Abtrennung von Dialkylcarbonat und $H_2O$, gegebenenfalls auch von $CO_2$, zurückgeführt werden.

Das erfindungsgemäße Verfahren kann in den verschiedenen bekannten Reaktortypen durchgeführt werden. Man kann beispielsweise in einem Rührwerkskessel mit Begasungsrührer arbeiten. Man kann diskontinuierlich, aber auch kontinuierlich arbeiten. Für die kontinuierliche Durchführung eignen sich auch Reaktionskessel-Kaskaden mit beispielsweise 3 - 5 Reaktoren. Für das Verfahren ist aber auch eine ein- oder mehrstufige Blasensäule geeignet.

Die Gasbelastung kann in weiten Grenzen variiert werden, je nach Druck und Temperatur, so daß Raumzeitausbeuten zwischen 10 und 200 g/l.h erreicht werden können.

Die Reaktionswärme kann durch Kühlaggregate entfernt werden. In einer besonderen Durchführungsform arbeitet man jedoch in einem sogenannten Siedereaktor, bei dem die Reaktionswärme durch verdampfendes Produkt abgeführt wird. So wird beispielsweise bei Flüssigeinspeisung von Alkanol die Reaktionswärme durch das Verdampfen des Alkanols aufgenommen; Kühlaggregate am Reaktor sind dabei nicht erforderlich. Der Austrag der Reaktionsprodukte Dialkylcarbonat und Wasser aus dem Reaktor erfolgt hierbei durch den Gasstrom. Die Konzentration der Stoffe im Gasstrom ist vom Druck und Temperatur abhängig. Es kann daher, insbesondere bei höherem Druck, vorteilhaft sein, kurzzeitig zu entspannen und anschließend wieder unter Druck zu arbeiten (sog. Pressure Swing-Technik). So kann man beispielsweise bei der Herstellung von Dimethylcarbonat so verfahren, daß man 1 min bis 10 min bei höherem Druck, beispielsweise bei 25 bis 50 bar, arbeitet und anschließend den Systemdruck auf etwa 10 bis 1 bar reduziert. Dabei bleibt die Schmelze vollständig in der Reaktionszone und die Organika, z.B. Dimethylcarbonat und Methanol, destillieren praktisch vollständig aus dem Reaktor aus. Diese taktweise Änderung des Systemsdruckes, die die Abtrennung der Reaktionsprodukte erleichtert, ist für das erfindungsgemäße Verfahren besonders geeignet.

Die Lebensdauer der Salzschmelze ist lang. Bei kontinuierlicher Fahrweise kann man einen Teilstrom z.B. 0,01 - 5 % der Salzschmelze pro Stunde herauspumpen und separat regenerieren. Die Regeneration kann durch behutsames Abbrennen mit Luft oder durch Auflösen in Wasser, Extraktion der wässrigen Phase und Eindampfen des Raffinates bis zur Trockne erfolgen.

3

Als Werkstoffe für die Reaktoren eignen sich beispielsweise korrosionsbeständige Edelstähle, Stahl-Emaille, Glas oder Sondermetalle wie Tantal.

Beispiel 1

In einem 1000 ml mit Ta-Einsatz versehenen Stahl-Rührwerksbehälter, ausgestattet mit Begasungsrührer und Druckhaltung, legte man 376 ml eines bei 150°C geschmolzenen Salzgemisches von 72 % Cu-I-chlorid und 28 % KCl vor. Dazu dosierte man 250 g/h Methanol, 24 l/h CO und 57,0 l/h Luft.

Bei einem Umsatz von 9,75 % Methanol erhielt man Dimethylcarbonat mit einer Raumzeitausbeute von 54,2 g/l.h mit einer Selektivität von 85,7 %.

Beispiel 2

In der gleichen Apparatur wie in Beispiel 1 erhielt man bei rhythmischer Entspannung auf 5 bar im Abstand von ca. 5 min folgende Ergebnisse:

| T °C | P bar | $O_2$ Vol.-% | Umsatz $CH_3OH$ % | SEL DMC % | SEL Formal % | SEL $CH_3Cl$ % |
|------|-------|--------------|-------------------|-----------|--------------|----------------|

Pressure Swing-Technik:

| T °C | P bar | $O_2$ Vol.-% | Umsatz $CH_3OH$ % | SEL DMC % | SEL Formal % | SEL $CH_3Cl$ % |
|------|-------|--------------|-------------------|-----------|--------------|----------------|
| 150 | 25 | 4,43 | 9,75 | 85,7 | 12,7 | 1,6 |
| 150 | 25 | 2,48 | 7,50 | 94,7 | 4,8 | 0,5 |
| 150 | 25 | 1,32 | 2,20 | 98,7 | 1,3 | 0 |

SEL = Selektivität (...zu...)

DMC = Dimethylcarbonat

Formal = Dimethylformal

Beispiel 3

In einem 1000 l mit Ta-Einsatz versehenen Stahlbehälter, ausgestattet mit Ein- und Ausgangsleitung, Druckhaltung und Temperaturmessung, legte man 94 ml eines bei 150°C geschmolzenen Salzgemisches von 72 % Cu-I-chlorid und 28 % KCl vor. Dazu dosierte man bei 50 bar Gesamtdruck 380 g/h Methanol, 36 l/h CO und 43 l/h Luft. Nach Entspannung erhielt man bei einem Umsatz von 7,2 % Methanol Dimethylcarbonat mit einer Raumzeitausbeute von 210 g/(l.h) und einer Selektivität von 96,5 %.

**Patentansprüche**

1. Verfahren zur Herstellung von $C_1$-$C_4$-Dialkylcarbonaten, dadurch gekennzeichnet, daß man $C_1$-$C_4$-Alkanole, CO und $O_2$ in einer Cu-salze enthaltenden Salzschmelze bei 120-300°C umsetzt.

2. Verfahren nach Anspruch 1, dadurch gekennzeichnet, daß man Methanol einsetzt.

3. Verfahren nach Anspruch 1, dadurch gekennzeichnet, daß man als Cu-Salze Cu-I- oder Cu-II-salze oder Gemische von Cu-I- und Cu-II-salzen in Form der Halogenide, der Cyanide, Sulfate, Nitrate, Nitrite, Carbonate, Acetate, Formiate, Oxalate, Alkoholate oder in Form von Komplexen-verbindungen einsetzt.

4. Verfahren nach Anspruch 1 und 3, dadurch gekennzeichnet, daß man als Cu-Salze die Cu-chloride einsetzt.

**5.** Verfahren nach Anspruch 1, dadurch gekennzeichnet, daß man Salzschmelzen verwendet, die neben Cu-Salzen Halogenide der 1.-3. Haupt- und/oder Nebengruppen enthalten.

**6.** Verfahren nach Anspruch 5, dadurch gekennzeichnet, daß man als Salzschmelze ein Gemisch von Cu-I-chlorid und KCl verwendet.

**7.** Verfahren nach Anspruch 1, dadurch gekennzeichnet, daß man bei Drücken von 5 - 50 bar arbeitet.

**8.** Verfahren nach Anspruch 1, dadurch gekennzeichnet, daß man ein Molverhältnis von Alkanol zu CO und $O_2$ von 1 : (1-0,01) : (1-0,01) wählt.

**9.** Verfahren nach Anspruch 1, dadurch gekennzeichnet, daß man die Reaktion in mit Begasungsrührern versehenen Reaktionskesseln oder in einer ein- oder mehrstufigen Blasensäule durchführt.

**10.** Verfahren nach Anspruch 1, dadurch gekennzeichnet, daß man den Systemdruck taktweise ändert (Pressure Swing-Technik).

## Claims

**1.** Process for the preparation of $C_1$-$C_4$-dialkyl carbonates, characterised in that $C_1$-$C_4$-alkanols, CO and $O_2$ are reacted at 120-300°C in a salt melt containing Cu salts.

**2.** Process according to Claim 1, characterised in that methanol is employed.

**3.** Process according to Claim 1, characterised in that Cu(I) or Cu(II) salts or mixtures of Cu(I) and Cu(II) salts in the form of the halides or the cyanides, sulphates, nitrates, nitrites, carbonates, acetates, formates, oxalates or alcoholates, or in the form of complex compounds, are employed as the Cu salts.

**4.** Process according to Claim 1 and 3, characterised in that Cu chlorides are employed as the Cu salts.

**5.** Process according to Claim 1, characterised in that salt melts which contain, in addition to Cu salts, halides of main and/or sub-groups 1-3 are used.

**6.** Process according to Claim 5, characterised in that a mixture of Cu(I) chloride and KCl is used as the salt melt.

**7.** Process according to Claim 1, characterised in that it is carried out under pressures of 5-50 bar.

**8.** Process according to Claim 1, characterised in that a molar ratio of alkanol to CO and $O_2$ of 1:(1-0.01):(1-0.01) is chosen.

**9.** Process according to Claim 1, characterised in that the reaction is carried out in reaction kettles provided with gassing stirrers or in a one- or multi-stage bubble column.

**10.** Process according to Claim 1, characterised in that the system pressure is changed cyclically (pressure swing technique).

## Revendications

**1.** Procédé pour la préparation des carbonates de dialkyle en $C_1$-$C_4$, caractérisé en ce que l'on fait réagir les alcanols en $C_1$-$C_4$, CO et $O_2$ dans une masse de sels fondue contenant des sels de cuivre à des températures de 120 à 300°C.

**2.** Procédé selon revendication 1, caractérisé en ce que l'on met en oeuvre du méthanol.

**3.** Procédé selon revendication 1, caractérisé en ce qu'on utilise en tant que sels de cuivre des sels cuivreux ou des sels cuivriques ou des mélanges de sels cuivreux et de sels cuivriques sous la forme des halogénures, des cyanures, des sulfates, des nitrates, des nitrites, des carbonates, des acétates, des formiates, des oxalates, des alcoolates ou sous la forme de complexes.

4. Procédé selon les revendications 1 et 3, caractérisé en ce que l'on utilise des chlorures de cuivre en tant que sels de cuivre.

5. Procédé selon revendication 1, caractérisé en ce que l'on utilise des masses de sels fondues contenant en plus des sels de cuivre des halogénures des métaux du premier au troisème groupe principal et/ou du premier au troisième sous-groupe.

6. Procédé selon revendication 5, caractérisé en ce que la masse de sels consiste en un mélange de chlorure cuivreux et de KCl.

7. Procédé selon revendication 1, caractérisé en ce que l'on opère à des pressions de 5 à 50 bar.

8. Procédé selon revendication 1, caractérisé en ce que l'on observe des proportions molaires alcanol/CO/$O_2$ de 1 : 1 à 0,01 : 1 à 0,01.

9. Procédé selon revendication 1, caractérisé en ce que l'on réalise la réaction dans des réacteurs équipés d'agitateurs à injection de gaz ou dans une colonne à bulles à un ou plusieurs étages.

10. Procédé selon revendication 1, caractérisé en ce que l'on fait varier périodiquement la pression dans l'installation (technique "Pressure Swing").